# EUROPEAN PATENT SPECIFICATION

(11) **EP 4 408 520 B1**
(45) Date of publication and mention of the grant of the patent: **10.06.2026**
(21) Application number: 22772992.8
(22) Date of filing: 07.09.2022
(51) Int. Cl.: A61N 1/372, A61B 5/00, A61B 5/11, G16H 40/67, G16H 50/20, G16H 50/80, H04W 4/80, A61N 1/36, A61N 1/37, H04W 4/02

(54) **COMPUTER IMPLEMENTED METHOD AND SYSTEM FOR CONTROLLING AN IMPLANTABLE MEDICAL DEVICE**
COMPUTERIMPLEMENTIERTES VERFAHREN UND SYSTEM ZUR STEUERUNG EINER IMPLANTIERBAREN MEDIZINISCHEN VORRICHTUNG
PROCÉDÉ ET SYSTÈME MIS EN OEUVRE PAR ORDINATEUR PERMETTANT DE COMMANDER UN DISPOSITIF MÉDICAL IMPLANTABLE

(30) Priority: 27.09.2021 US 202163248626 P; 13.10.2021 EP 21202406
(43) Date of publication of application: 07.08.2024
(73) Proprietor: BIOTRONIK SE & Co. KG, 12359 Berlin (DE)
(72) Inventor: BECKER, Andreas, Tigard, Oregon 97224 (US); MUESSIG, Dirk, West Linn, Oregon 97068 (US)
(74) Representative: Biotronik Corporate Services SE
(86) International application number: PCT/EP2022/074856
(87) International publication number: WO 2023/046479

(56) References cited:
- WO-A1-2021/188043
- US-A1- 2014 058 383
- US-A1- 2020 146 552
- US-A1- 2021 020 294
- PANICACCI SILVIA ET AL: "Empowering Home Health Monitoring of Covid-19 Patients with Smartwatch Position and Fitness Tracking", 2021 IEEE 34TH INTERNATIONAL SYMPOSIUM ON COMPUTER-BASED MEDICAL SYSTEMS (CBMS), IEEE, 7 June 2021 (2021-06-07), pages 348 - 353, XP033939822, DOI: 10.1109/CBMS52027.2021.00109

## Description

The invention relates to a computer implemented method for controlling an implantable medical device. Furthermore, the invention relates a system for controlling an implantable medical device.

Situations such as the COVID-19 pandemic show that an early indication of systemic inflammations is essential to lower the spread and to increase therapy success. Symptoms of COVID-19 are fever, dry cough, fatigue, sputum production, loss of smell, and shortness of breath, which are usually developed between 2 and 14 days after exposure. However, examples show that due to inflammatory processes in the lung the blood oxygen content in the blood may drop slowly, before symptoms are ongoing. This drop is not easily recognized when a patient is in a healthy condition, because it is partly compensated by an increased heart rate in order to fulfill the demand.

One can assume that patients wearing active medical implants like devices in the field of CRM (Cardiac Rhythm Management) such as implantable pacemakers, implantable cardioverter/defibrillators, CRT (Cardiac Resynchronization Therapy) devices or cardiac monitors pertain to a high-risk group of patients when it comes to an increased heart rate. This condition can be life threatening and needs immediate attention and therapy. Other groups of high risk include patients wearing implantable drug pumps for therapy of chronic diseases like for example diabetes, or certain neurological stimulators, like Deep Brain Stimulation (DBS) devices, Vagal Nerve Stimulation (VNS) devices or Spinal Cord Stimulation (SCS) devices.

US 2020/146552 A1 shows systems, devices, and methods for monitoring patients using information corresponding to their location. US 2021/020294 A1 shows methods, devices and systems for holistic management of patients with chronic disease based on an integrated analysis of dissimilar patient data from unrelated data sources.

Currently, there are no existing solutions to address the problem of identifying early indications of systemic inflammations for patients using implantable medical devices.

It is therefore an object of the present invention to provide an improved method and system for controlling an implantable medical device capable of identifying early indications of systemic inflammations such that immediate attention and therapy can be provided.

The object is solved by a computer implemented method for controlling an implantable medical device having the features of claim 1.

The object is furthermore solved by a system for controlling an implantable medical device having the features of claim 13.

Moreover, the object is solved by a computer program of claim 14 and a computer-readable data carrier having the features of claim 15.

The present invention provides a computer implemented method for controlling an implantable medical device.

The method provides the step of detecting a geographic location of the implantable medical device and/or a proximity of the implantable medical device to an object and/or a person.

Furthermore, the method provides the step of at least temporarily activating and/or deactivating at least one diagnostic function of the implantable medical device and/or at least temporarily changing a transmission frequency and/or a selection of at least one medical parameter of a patient detected by the implantable medical device if the detected geographic location of the implantable medical device and/or the proximity of the implantable medical device to the object and/or person meets a predetermined condition.

In addition, the present invention provides a system for controlling an implantable medical device.

The system comprises a patient device configured to detect a geographic location of the implantable medical device and/or a proximity of the implantable medical device to an object and/or person.

Furthermore, the system comprises the implantable medical device, wherein the patient device is further configured to at least temporarily activate and/or deactivate at least one diagnostic function of the implantable medical device and/or at least temporarily change a transmission frequency and/or a selection of at least one medical parameter of a patient detected by the implantable medical device if the detected geographic location of the implantable medical device and/or the proximity of the implantable medical device to the object and/or person meets a predetermined condition.

Instead of the Patient Device activates/deactivates diagnostics, or changes the transmission frequency and/or a selection of at least one medical parameter, another alternative or additional possibility is that a remote server activates/deactivates diagnostics the implantable medical device (via the patient device as hub) and/or starts to more frequently interrogate the defined parameters from the implantable device. This can be done by an algorithm. E.g. if a connectivity between Patient Device and server is established, interrogation would be the preferred communication. If no connection to/from the remote server can be established, the Patient Device triggers implantable medical device as described above.

Moreover, the present invention provides a computer program with program code to perform the method according to the present invention when the computer program is executed on a computer. In addition, the present invention provides a computer readable data carrier with program code of a computer program to perform the method according to the present invention when the computer program is executed on a computer.

It is an idea of the present invention to provide a situation dependent, temporary diagnostic or therapy feature to a specific patient population, which leads to an enhanced patient support while saving lifetime of an implantable medical device.

Further, it is thus possible to monitor high risk patients closely, after they have gotten in contact with infected persons or travel in high-risk areas, and to react quickly, if they show a change of parameters characteristic for certain infections or to SIRS (Systemic Inflammatory Response Syndrome) in general. Most common symptoms of SIRS are the following:
- Body temperature less than 36°C (96.8°F) or greater than 38°C (100.4°F)
- Heart rate greater than 90 beats per minute
- Tachypnea (high respiratory rate), with greater than 20 breaths per minute; or, an arterial partial pressure of carbon dioxide less than 4.3 kPa (32 mmHg)
- White blood cell count less than 4000 cells/mm³ (4 x 109 cells/L) or greater than 12,000 cells/mm³ (12 x 109 cells/L); or the presence of greater than 10% immature neutrophils (band forms). Band forms greater than 3% is called bandemia or a "left-shift."

When two or more of these criteria are met with or without evidence of infection, patients may be diagnosed with "SIRS."

Additionally or alternatively, the system and/or method according to the invention may detect other symptoms as less body movement and/or specific posture patterns like lying down for a long time, which can mostly accompany one or more of the four indications of SIRS above.

According to an aspect of the invention, the geographic location of the implantable medical device is detected by a patient device, in particular a handheld or wearable device, in particular preferably a smartphone, a smartwatch and/or a tablet computing device, wirelessly communicating with the implantable medical device, wherein the patient device determines the geographic location of the implantable medical device using GNSS ("Global Navigation Satellite System") and/or mobile data.

Since the above-mentioned devices are customarily equipped with a GNSS sensor and/or can determine their current position based on mobile signals, the position of the implantable medical device can consequently be also determined using the patient device.

According to a further aspect of the invention, if the predetermined condition is met, the patient device controls the implantable medical device, in particular by wireless communication, in particular preferably by Bluetooth low energy and/or Mics-Band communication, to at least temporarily activate and/or deactivate the at least one diagnostic function of the implantable medical device and/or to at least temporarily change the transmission frequency and/or the selection of the at least one medical parameter of a patient detected by the implantable medical device.

Thus, the battery intensive detecting of the geographic location of the implantable medical device and/or the proximity of the implantable medical device to an object and/or person as well as the detection, if the predetermined condition is met, is thus performed by the patient device, for which battery/energy usage is no issue. This in turn conserves energy of the implantable medical device.

According to a further aspect of the invention, the predetermined condition is met if the detected geographic location of the implantable medical device is classified as a high-risk area or poses a patient specific risk, in particular dependent on a specified patient condition and/or if a proximity of the implantable medical device to a high-risk object and/or a high-risk person is detected. If one of the above-mentioned conditions is met, the implantable medical device can thus advantageously activate a diagnostic function in order to check if the medical parameters of the patient deviate from an expected range.

According to a further aspect of the invention, geographic location data of the implantable medical device and/or proximity data of the implantable medical device to the object and/or person detected by the patient device is compared to data on high-risk areas and/or patient specific risks stored in a database hosted on the patient device, the implantable medical device and/or a remote server accessible by the patient device via a network in order to determine if the predetermined condition is met. Said data comparison advantageously enables a verification of whether or not the patient was subjected to a potential risk.

According to a further aspect of the invention, the diagnostic function of the implantable medical device is selected by a health care provider, in particular a physician, or by an algorithm using medical data of the patient stored in the patient device, the implantable medical device and/or the remote server accessible by the patient device via the network. This way the most appropriate diagnostic function of the implantable medical device can advantageously be selected.

According to a further aspect of the invention, the content of the database is synchronized between the remote server and the implantable medical device and/or the patient device at predetermined intervals and/or when the database is updated. The implantable medical device thus always has access to current data on high-risk areas and/or patient specific risks.

According to a further aspect of the invention, if the predetermined condition is met, the diagnostic function of the implantable medical device is activated and/or deactivated and/or the transmission frequency and/or the selection of the at least one medical parameter of the patient detected by the implantable medical device is changed for a predetermined period of time after which the previous setting is automatically resumed or until receipt of a user generated cancellation request. The activated diagnostic function of the implantable medical device is thus run for a time period suitable to the detected risk. The cancellation request may include/cause (that) the remote server is activating/deactivating diagnostic function and/or changing the transmission frequency and/or the selection of the at least one medical parameter.

According to a further aspect of the invention, the diagnostic function of the implantable medical device is an ECG, a temperature, impedance, respiratory function, sound, posture and/or movement measurement and/or tracking by sensors of the implantable medical device. The sound measurement can e.g. be used to identify heart tones of the patient and to determine whether or not the measured heart tones are within an expected range. Activating diagnose function can additionally include activation of certain (self-learning) algorithms in the implant, the patient remote and/or the remote server for analyzing the correlation of the at least a subset of the above mentioned measurement, or additional values like publicly available environmental data or subjective data from the patient (e.g. via answering surveys displayed on the patient device).

According to a further aspect of the invention, the at least one diagnostic function of the implantable medical device is activated and/or deactivated, and/or the transmission frequency and/or the selection of the at least one medical parameter of the patient detected by the implantable medical device is changed for a period of time defined by the patient and/or the health care provider, in particular a travel time period and/or a quarantine duration and/or for a time period automatically set by the implantable medical device or the patient device, in particular an incubation time. The measurement time can thus advantageously be adapted to an individual situation of the patient.

According to a further aspect of the invention, the at least one medical parameter of the patient is surveilled for a predetermined period of time, wherein if the at least one medical parameter of the patient is outside a predefined range a message is sent from the patient device to the remote server accessible by the health care provider and/or a therapeutic function of the implantable medical device is activated. The surveillance of the measured medical parameter is thus advantageously performed at a time when needed in order to reduce a potential health risk for the patient.

According to a further aspect of the invention, if a proximity of the implantable medical device within a predefined range, in particular within 1.5m, to an infected person is detected, by means of proximity tracing using Bluetooth, GNSS and/or mobile data, the predetermined condition is met and/or an alert is sent from the patient device to the remote server accessible by the health care provider. Proximity tracing or in other words contact tracing can thus advantageously be used to determine whether the patient was subjected to a health risk.

The herein described features of the computer implemented method for controlling an implantable medical device are also disclosed for the system for controlling an implantable medical device and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a flowchart of a computer implemented method for controlling an implantable medical device according to a preferred embodiment of the invention; and
- Fig. 2: shows a schematic view of a system for controlling an implantable medical device according to the preferred embodiment of the invention.

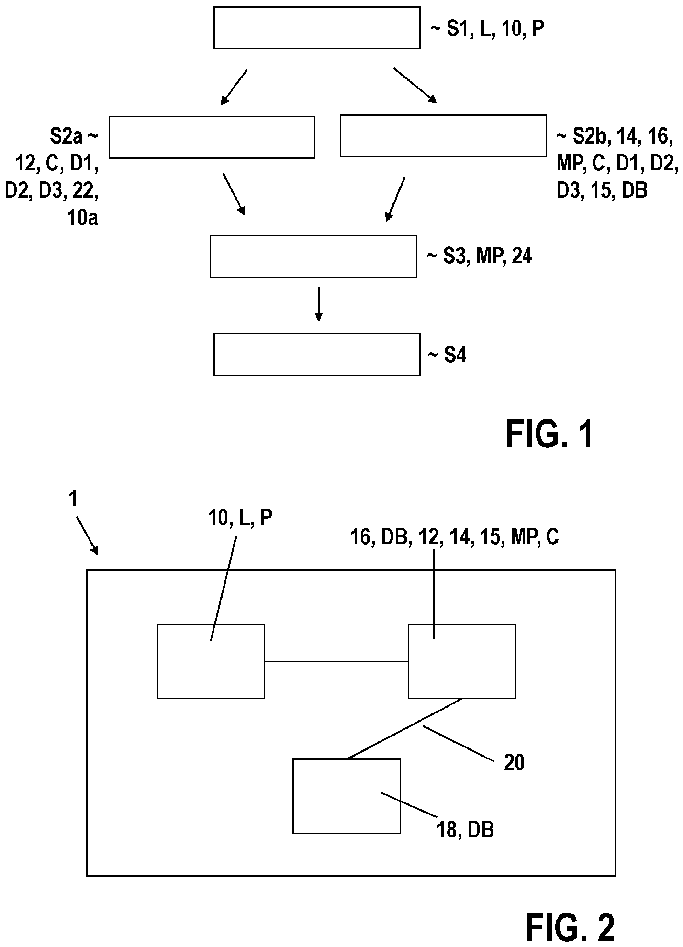

The computer implemented method for controlling an implantable medical device 10 shown in Fig. 1 comprises detecting S1 a geographic location L of the implantable medical device 10 and/or a proximity P of the implantable medical device 10 to an object and/or person.

Furthermore, the method comprises at least temporarily activating and/or deactivating S2a at least one diagnostic function 12 of the implantable medical device 10 and/or at least temporarily changing S2b a transmission frequency 14 and/or a selection 15 of at least one medical parameter MP of a patient detected by the implantable medical device 10 if the detected geographic location L of the implantable medical device 10 and/or the proximity P of the implantable medical device 10 to the object and/or person meets a predetermined condition C.

The geographic location L of the implantable medical device 10 is detected by a patient device 16, in particular a handheld or wearable device, in particular preferably a smartphone, a smartwatch and/or a tablet computing device, wirelessly communicating with the implantable medical device 10, wherein the patient device 16 determines the geographic location L of the implantable medical device 10 using GNSS and/or mobile data.

If the predetermined condition C is met, the patient device 16 controls the implantable medical device 10, in particular by wireless communication, in particular preferably by Bluetooth low energy and/or Mics-Band communication, to at least temporarily activate and/or deactivate S2a the at least one diagnostic function 12 of the implantable medical device 10 and/or to at least temporarily change S2b the transmission frequency 14 and/or the selection 15 of the at least one medical parameter MP of a patient detected by the implantable medical device 10.

The predetermined condition C is met if the detected geographic location L of the implantable medical device 10 is classified as a high-risk area or poses a patient specific risk, in particular dependent on a specified patient condition and/or if a proximity P of the implantable medical device 10 to a high-risk object and/or a high-risk person is detected.

Geographic location data D1 of the implantable medical device 10 and/or proximity data D2 of the implantable medical device 10 to the object and/or person detected by the patient device 16 is compared to data D3 on high-risk areas and/or patient specific risks stored in a database DB hosted on the patient device 16, the implantable medical device 10 and/or a remote server 18 (see Fig. 2) accessible by the patient device 16 via a network 20 (see Fig. 2) in order to determine if the predetermined condition C is met.

I.e., it can be determined if the current location is in an area of high risk such as a pandemic area or an area of war, or in other environments, which would create a high risk to the patient, e.g. high altitude, for which a specified close diagnosis or patient surveillance may be needed.

The database can be located remotely from the personal location device and the implant in an internal remote server like a home monitoring service center, or in an external data base, e.g. pertinent databases from organizations like governments, UN/WHO, or maps.

The diagnostic function 12 of the implantable medical device 10 is selected by a health care provider, in particular a physician, or by an algorithm using medical data 22 of the patient stored in the patient device 16, the implantable medical device 10 and/or the remote server 18 accessible by the patient device 16 via the network 20.

The content of the database DB is synchronized between the remote server 18 and the implantable medical device 10 and/or the patient device 16 at predetermined intervals and/or when the database DB is updated. The patient's health care provider may be informed about this circumstance of entering a high-risk country/area.

If the predetermined condition C is met, the diagnostic function 12 of the implantable medical device 10 is activated and/or deactivated and/or the transmission frequency 14 and/or the selection 15 of the at least one medical parameter MP of the patient detected by the implantable medical device 10 is changed for a predetermined period of time after which the previous setting is automatically resumed or until receipt of a user generated cancellation request.

The diagnostic function 12 of the implantable medical device 10 is an ECG, a temperature, impedance, respiratory function, sound and/or movement/posture measurement and/or tracking or recognition of specific signal patterns, like surveillance of behavior of SIRS parameters (Systemic Inflammatory Response Syndrome), etc. by sensors 10a of the implantable medical device 10. Activating a diagnostic or sensing feature means that functions will be activated upon determination of a/the geographic location L, which are usually not active or rarely active in the implantable medical device under normal conditions. Activating diagnose function can additionally include activation of certain (self-learning) algorithms in the implant, the patient remote and/or the remote server 18 for analyzing the correlation of the at least a subset of the above mentioned measurement, or additional values like publicly available environmental data or subjective data from the patient (e.g. via answering surveys displayed on the patient device 16).

These functions can be stored and/or pre-installed in the implantable medical device 10, or - upon determination of the geographic location L - can be selected for additional installation on the implantable medical device, either via remote programming from the remote server 18 (where the function is stored and assigned to the determined geographic location L), or from implant associated personal locating device, e.g. the patient device 16, to which the function is either downloaded on demand or pre-installed.

The selection 15 can be made by a physician or by a suitable algorithm considering all available data. In a further alternative or combination, specific therapeutic functions can be activated in the same manner, like medication or stimulation regimes, or similar.

Further, as a precautionary action, therapy proposals can be displayed on the implant associated personal location device, e.g. the administering of OTC medication or the like. Additionally, electronic surveys can be sent to the implant associated location device for retrieving subjective patient data.

The at least one diagnostic function 12 of the implantable medical device 10 is activated and/or deactivated, and/or the transmission frequency 14 and/or the selection 15 of the at least one medical parameter MP of the patient detected by the implantable medical device 10 is changed for a period of time defined by the patient and/or the health care provider, in particular a travel time period and/or a quarantine duration and/or for a time period automatically set by the implantable medical device 10 or the patient device 16, in particular an incubation time or alternatively, an unlimited or undefined period of time, which can be de-activated on health care providers discretion only.

The activated diagnostic function 12 of the implantable medical device 10 can then e.g., be automatically or manually (by physician or patient) deactivated after return from the geographic location L.

Another additional or alternative function can be a change in the telemetry function of the device, which routinely transmits sensed or functional data from the implant directly or over several hops to an internal service center. This internal service center may be located remotely, e.g. on a remote server 18. Dependent on the analysis (e.g. by a suitable algorithm) the change can concern the frequency of these messages (e.g. several times a day, hourly, real-time) and/or the latency, the resolution and accuracy.

The patient's health care provider may be informed about activating diagnostic/sensing functions, also. An entry into patient's Electronic Health Record (EHR) can be optionally created by a suitable algorithm, thereby creating all the necessary information (survey data included).

The at least one medical parameter MP of the patient is surveilled S3 for a predetermined period of time, wherein if the at least one medical parameter MP of the patient is outside a predefined range a message is sent from the patient device 16 to the remote server 18 accessible by the health care provider and/or a therapeutic function 24 of the implantable medical device 10 is activated.

If a/the proximity P of the implantable medical device 10 within a predefined range, in particular within 1.5m, to an infected person is detected, by means of proximity P tracing using Bluetooth, GNSS and/or mobile data, the predetermined condition C is met and/or an alert is sent S4 from the patient device 16 to the remote server 18 accessible by the health care provider.

The data derived from the activated diagnostic function 12 can be transmitted to the patient's health care provider for further consideration. This transmission can occur on a regular basis, or after reaching a certain threshold, milestone or signal pattern. The health care provider can then perform usual steps like getting in contact with the patient. Additionally, or alternatively a/the therapeutic function 24 can be started, or therapy proposals can be sent to the implant associated patient locating device, e.g. the patient device 16, or electronic prescriptions can be established for further medication therapy.

Fig. 2 shows a schematic view of a system for controlling an implantable medical device according to the preferred embodiment of the invention.

The system 1 comprises a patient device 16 configured to detect a geographic location L of the implantable medical device 10 and/or a proximity P of the implantable medical device 10 to an object and/or person.

Furthermore, the system 1 comprises the implantable medical device 10, wherein the patient device 16 is further configured to at least temporarily activate and/or deactivate at least one diagnostic function 12 of the implantable medical device 10 and/or at least temporarily change a transmission frequency 14 and/or a selection 15 of at least one medical parameter MP of a patient detected by the implantable medical device 10 if the detected geographic location L of the implantable medical device 10 and/or the proximity P of the implantable medical device 10 to the object and/or person meets a predetermined condition C.

### Further embodiments:

A computer implemented method for controlling a medical device implanted into the human or animal body. The method comprises detecting S1 a geographic location L of the implantable medical device 10 and/or a proximity P of the implantable medical device 10 to an object and/or person.

Furthermore, the method comprises at least temporarily activating and/or deactivating S2a at least one diagnostic function 12 of the implantable medical device 10 and/or at least temporarily changing S2b a transmission frequency 14 and/or a selection 15 of at least one medical parameter MP of a patient detected by the implantable medical device 10 if the detected geographic location L of the implantable medical device 10 and/or the proximity P of the implantable medical device 10 to the object and/or person meets a predetermined condition C.

### Reference Signs

- 1: system
- 10: implantable medical device
- 10a: sensors
- 12: diagnostic function
- 14: transmission frequency
- 15: selection
- 16: patient device
- 18: remote server
- 20: network
- 22: medical data
- 24: therapeutic function
- C: predetermined condition
- D1: geographic location data
- D2: proximity data
- D3: data
- DB: database
- L: geographic location
- MP: medical parameter
- P: proximity
- S1, S2a, S2b, S3, S4: method steps

## Claims

1. Computer implemented method for controlling an implantable medical device (10) comprising the steps of:
detecting (S1) by a patient device (16) a geographic location (L) of the implantable medical device (10) and/or a proximity (P) of the implantable medical device (10) to an object and/or person; and
controlling the implantable medical device (10) by the patient device (16) by Bluetooth low energy and/or MICS band communication to at least temporarily activating and/or deactivating (S2a) at least one diagnostic function (12) of the implantable medical device (10) and/or at least temporarily changing (S2b) a transmission frequency (14) and/or a selection (15) of at least one medical parameter (MP) of a patient detected by the implantable medical device (10) if the detected geographic location (L) of the implantable medical device (10) and/or the proximity (P) of the implantable medical device (10) to the object and/or person meets a predetermined condition (C).

2. Computer implemented method of claim 1, wherein the patient device (16) is a handheld or wearable device, in particular preferably a smartphone, a smartwatch and/or a tablet computing device.

3. Computer implemented method of claim 1 or 2, wherein the patient device wirelessly communicates with the implantable medical device (10), wherein the patient device (16) determines the geographic location (L) of the implantable medical device (10) using GNSS and/or mobile data.

4. Computer implemented method of any one of the preceding claims, wherein the predetermined condition (C) is met if the detected geographic location (L) of the implantable medical device (10) is classified as a high-risk area or poses a patient specific risk, in particular dependent on a specified patient condition and/or if a proximity (P) of the implantable medical device (10) to a high-risk object and/or a high-risk person is detected.

5. Computer implemented method of claim 4, wherein geographic location data (D1) of the implantable medical device (10) and/or proximity data (D2) of the implantable medical device (10) to the object and/or person detected by the patient device (16) is compared to data (D3) on high-risk areas and/or patient specific risks stored in a database (DB) hosted on the patient device (16), the implantable medical device (10) and/or a remote server (18) accessible by the patient device (16) via a network (20) in order to determine if the predetermined condition (C) is met.

6. Computer implemented method of claim 5, wherein the diagnostic function (12) of the implantable medical device (10) is selected by a health care provider, in particular a physician, or by an algorithm using medical data (22) of the patient stored in the patient device (16), the implantable medical device (10) and/or the remote server (18) accessible by the patient device (16) via the network (20).

7. Computer implemented method of claim 5 or 6, wherein the content of the database (DB) is synchronized between the remote server (18) and the implantable medical device (10) and/or the patient device (16) at predetermined intervals and/or when the database (DB) is updated.

8. Computer implemented method of any one of the preceding claims, wherein if the predetermined condition (C) is met, the diagnostic function (12) of the implantable medical device (10) is activated and/or deactivated and/or the transmission frequency (14) and/or the selection (15) of the at least one medical parameter (MP) of the patient detected by the implantable medical device (10) is changed for a predetermined period of time after which the previous setting is automatically resumed or until receipt of a user generated cancellation request.

9. Computer implemented method of any one of the preceding claims, wherein the diagnostic function (12) of the implantable medical device (10) is a temperature, impedance, respiratory function, sound, posture and/or movement measurement and/or tracking by sensors (10a) of the implantable medical device (10).

10. Computer implemented method of any one of the preceding claims, wherein the at least one diagnostic function (12) of the implantable medical device (10) is activated and/or deactivated, and/or the transmission frequency (14) and/or the selection (15) of the at least one medical parameter (MP) of the patient detected by the implantable medical device (10) is changed for a period of time defined by the patient and/or the health care provider, in particular a travel time period and/or a quarantine duration and/or for a time period automatically set by the implantable medical device (10) or the patient device (16), in particular an incubation time.

11. Computer implemented method of any one of claims 6 to 10, wherein the at least one medical parameter (MP) of the patient is surveilled (S3) for a predetermined period of time, wherein if the at least one medical parameter (MP) of the patient is outside a predefined range a message is sent from the patient device (16) to the remote server (18) accessible by the health care provider and/or a therapeutic function (24) of the implantable medical device (10) is activated.

12. Computer implemented method of any one of the preceding claims, wherein if a proximity (P) of the implantable medical device (10) within a predefined range, in particular within 1.5m, to an infected person is detected, by means of proximity (P) tracing using Bluetooth, GNSS and/or mobile data, the predetermined condition (C) is met and/or an alert is sent (S4) from the patient device (16) to the remote server (18) accessible by the health care provider.

13. System (1) for controlling an implantable medical device (10) comprising:
a patient device (16) configured to detect a geographic location (L) of the implantable medical device (10) and/or a proximity (P) of the implantable medical device (10) to an object and/or person; and
the implantable medical device (10), wherein the patient device (16) is further configured to control the implantable medical device (10) by Bluetooth low energy and/or MICS band communication to at least temporarily activate and/or deactivate at least one diagnostic function (12) of the implantable medical device (10) and/or at least temporarily change a transmission frequency (14) and/or a selection (15) of at least one medical parameter (MP) of a patient detected by the implantable medical device (10) if the detected geographic location (L) of the implantable medical device (10) and/or the proximity (P) of the implantable medical device (10) to the object and/or person meets a predetermined condition (C).

14. Computer program with program code to perform the method of any one of claims 1 to 12 when the computer program is executed by the system of claim 13.

15. Computer readable data carrier with program code of a computer program to perform the method of any one of claims 1 to 12 when the computer program is executed by the system of claim 13.

## Patentansprüche

1. Computerimplementiertes Verfahren zur Steuerung eines implantierbaren medizinischen Geräts (10), das die folgenden Schritte umfasst:
Erfassen (S1) eines geografischen Standorts (L) des implantierbaren medizinischen Geräts (10) und/oder einer Nähe (P) des implantierbaren medizinischen Geräts (10) zu einem Objekt und/oder einer Person durch ein Patientengerät (16); und
Steuern des implantierbaren medizinischen Geräts (10) durch das Patientengerät (16) mittels Bluetooth-Low-Energy- und/oder MICS-Band-Kommunikation, um mindestens vorübergehend mindestens eine Diagnosefunktion (12) des implantierbaren medizinischen Geräts (10) zu aktivieren und/oder zu deaktivieren (S2a) und/oder mindestens vorübergehend eine Übertragungsfrequenz (14) und/oder eine Auswahl (15) mindestens eines medizinischen Parameters (MP) eines Patienten, der von dem implantierbaren medizinischen Gerät (10) erfasst wird, wenn der erfasste geographische Standort (L) des implantierbaren medizinischen Gerätes (10) und/oder die Nähe (P) des implantierbaren medizinischen Gerätes (10) zu dem Objekt und/oder der Person eine vorbestimmte Bedingung (C) erfüllt.

2. Computerimplementiertes Verfahren nach Anspruch 1, wobei das Patientengerät (16) ein Handgerät oder tragbares Gerät ist, insbesondere vorzugsweise ein Smartphone, eine Smartwatch und/oder ein Tablet-Computergerät.

3. Computerimplementiertes Verfahren nach Anspruch 1 oder 2, wobei das Patientengerät drahtlos mit dem implantierbaren medizinischen Gerät (10) kommuniziert, wobei das Patientengerät (16) den geografischen Standort (L) des implantierbaren medizinischen Geräts (10) unter Verwendung von GNSS und/oder mobilen Daten bestimmt.

4. Computerimplementiertes Verfahren nach einem der vorstehenden Ansprüche, wobei die vorbestimmte Bedingung (C) erfüllt ist, wenn der erfasste geografische Standort (L) des implantierbaren medizinischen Geräts (10) als Hochrisikobereich klassifiziert wird oder ein patientenspezifisches Risiko darstellt, insbesondere abhängig von einem bestimmten Patientenstatus, und/oder wenn eine Nähe (P) des implantierbaren medizinischen Geräts (10) zu einem Hochrisikoobjekt und/oder einer Hochrisikoperson erkannt wird.

5. Computerimplementiertes Verfahren nach Anspruch 4, wobei geografische Standortdaten (D1) des implantierbaren medizinischen Geräts (10) und/oder Näherungsdaten (D2) des implantierbaren medizinischen Geräts (10) zu dem vom Patientengerät (16) erkannten Objekt und/oder der Person mit Daten (D3) zu Hochrisikobereichen und/oder patientenspezifischen Risiken verglichen werden, die in einer Datenbank (DB) gespeichert sind, die auf dem Patientengerät (16), dem implantierbaren medizinischen Gerät (10) und/oder einem Fernserver (18), auf den das Patientengerät (16) über ein Netzwerk (20) zugreifen kann, gespeichert sind, um festzustellen, ob die vorbestimmte Bedingung (C) erfüllt ist.

6. Computerimplementiertes Verfahren nach Anspruch 5, wobei die Diagnosefunktion (12) des implantierbaren medizinischen Geräts (10) von einem Gesundheitsdienstleister, insbesondere einem Arzt, oder durch einen Algorithmus unter Verwendung von medizinischen Daten (22) des Patienten ausgewählt wird, die in dem Patientengerät (16), dem implantierbaren medizinischen Gerät (10) und/oder dem Fernserver (18) gespeichert sind, und auf die das Patientengerät (16) über das Netzwerk (20) zugreifen kann.

7. Computerimplementiertes Verfahren nach Anspruch 5 oder 6, wobei der Inhalt der Datenbank (DB) zwischen dem Remote-Server (18) und dem implantierbaren medizinischen Gerät (10) und/oder dem Patientengerät (16) in vorbestimmten Intervallen und/oder bei einer Aktualisierung der Datenbank (DB) synchronisiert wird.

8. Computerimplementiertes Verfahren nach einem der vorstehenden Ansprüche, wobei, wenn die vorbestimmte Bedingung (C) erfüllt ist, die Diagnosefunktion (12) des implantierbaren medizinischen Geräts (10) aktiviert und/oder deaktiviert und/oder die Übertragungsfrequenz (14) und/oder die Auswahl (15) des mindestens einen medizinischen Parameters (MP) des Patienten, der von dem implantierbaren medizinischen Gerät (10) erfasst wird, für einen vorbestimmten Zeitraum geändert wird, nach dessen Ablauf die vorherige Einstellung automatisch wieder aufgenommen wird oder bis zum Empfang einer vom Benutzer generierten Stornierungsanforderung.

9. Computerimplementiertes Verfahren nach einem der vorstehenden Ansprüche, wobei die Diagnosefunktion (12) des implantierbaren medizinischen Geräts (10) eine Temperatur-, Impedanz-, Atemfunktions-, Geräusch-, Haltungs- und/oder Bewegungsmessung und/oder -verfolgung durch Sensoren (10a) des implantierbaren medizinischen Geräts (10) ist.

10. Computerimplementiertes Verfahren nach einem der vorstehenden Ansprüche, wobei die mindestens eine Diagnosefunktion (12) des implantierbaren medizinischen Geräts (10) aktiviert und/oder deaktiviert wird und/oder die Übertragungsfrequenz (14) und/oder die Auswahl (15) des mindestens einen medizinischen Parameters (MP) des Patienten, der von dem implantierbaren medizinischen Gerät (10) erfasst wird, für einen vom Patienten und/oder dem Gesundheitsdienstleister definierten Zeitraum, insbesondere einen Reisezeitraum und/oder eine Quarantänedauer und/oder für einen von dem implantierbaren medizinischen Gerät (10) oder dem Patientengerät (16) automatisch festgelegten Zeitraum, insbesondere eine Inkubationszeit, geändert wird.

11. Computerimplementiertes Verfahren nach einem der Ansprüche 6 bis 10, wobei der mindestens eine medizinische Parameter (MP) des Patienten für einen vorbestimmten Zeitraum überwacht wird (S3), wobei, wenn der mindestens eine medizinische Parameter (MP) des Patienten außerhalb eines vordefinierten Bereichs liegt, eine Nachricht von dem Patientengerät (16) an den Fernserver (18), auf den der Gesundheitsdienstleister zugreifen kann, und/oder eine therapeutische Funktion (24) des implantierbaren medizinischen Geräts (10) aktiviert wird.

12. Computerimplementiertes Verfahren nach einem der vorstehenden Ansprüche, wobei, wenn eine Nähe (P) des implantierbaren medizinischen Geräts (10) innerhalb eines vordefinierten Bereichs, insbesondere innerhalb von 1,5 m, zu einer infizierten Person mittels Näherungserkennung (P) unter Verwendung von Bluetooth, GNSS und/oder mobilen Daten eine vorbestimmte Bedingung (C) erfüllt ist und/oder eine Warnmeldung (S4) vom Patientengerät (16) an den für den Gesundheitsdienstleister zugänglichen Remote-Server (18) gesendet wird.

13. System (1) zur Steuerung eines implantierbaren medizinischen Geräts (10), umfassend:
ein Patientengerät (16), das so konfiguriert ist, dass es einen geografischen Standort (L) des implantierbaren medizinischen Geräts (10) und/oder eine Nähe (P) des implantierbaren medizinischen Geräts (10) zu einem Objekt und/oder einer Person erfasst; und
das implantierbare medizinische Gerät (10), wobei das Patientengerät (16) ferner dazu konfiguriert ist, das implantierbare medizinische Gerät (10) über Bluetooth Low Energy und/oder MICS-Band-Kommunikation zu steuern, um zumindest vorübergehend mindestens eine Diagnosefunktion (12) des implantierbaren medizinischen Geräts (10) zu aktivieren und/oder zu deaktivieren und/oder zumindest vorübergehend eine Übertragungsfrequenz (14) und/oder eine Auswahl (15) von mindestens ein en medizinischen Parametern (MP) eines Patienten, die von dem implantierbaren medizinischen Gerät (10) erfasst werden, vorübergehend zu ändern, wenn der erfasste geografische Standort (L) des implantierbaren medizinischen Geräts (10) und/oder die Nähe (P) des implantierbaren medizinischen Geräts (10) zu dem Objekt und/oder der Person eine vorbestimmte Bedingung (C) erfüllt.

14. Computerprogramm mit Programmcode zur Durchführung des Verfahrens nach einem der Ansprüche 1 bis 12, wenn das Computerprogramm durch das System nach Anspruch 13 ausgeführt wird.

15. Computerlesbarer Datenträger mit Programmcode eines Computerprogramms zur Durchführung des Verfahrens nach einem der Ansprüche 1 bis 12, wenn das Computerprogramm durch das System nach Anspruch 13 ausgeführt wird.

## Revendications

1. Procédé mis en œuvre par ordinateur pour commander un dispositif médical implantable (10) comprenant les étapes consistant à :
faire détecter (S1) par un dispositif patient (16) un emplacement géographique (L) du dispositif médical implantable (10) et/ou une proximité (P) du dispositif médical implantable (10) vis-à-vis d'un objet et/ou d'une personne ; et
faire commander le dispositif médical implantable (10) par le dispositif patient (16) par Bluetooth à basse consommation et/ou communication sur bande MICS pour activer et/ou désactiver (S2a) au moins temporairement au moins une fonction de diagnostic (12) du dispositif médical implantable (10) et/ou modifier (S2b) au moins temporairement une fréquence de transmission (14) et/ou une sélection (15) d'au moins un paramètre médical (MP) d'un patient détecté par le dispositif médical implantable (10) si l'emplacement géographique détecté (L) du dispositif médical implantable (10) et/ou la proximité (P) du dispositif médical implantable (10) vis-à-vis de l'objet et/ou de la personne répond à une condition prédéterminée (C).

2. Procédé mis en œuvre par ordinateur selon la revendication 1, dans lequel le dispositif patient (16) est un dispositif portable ou vestimentaire, en particulier de préférence un smartphone, une montre connectée et/ou un dispositif d'ordinateur-tablette.

3. Procédé mis en œuvre par ordinateur selon la revendication 1 ou 2, dans lequel le dispositif patient communique sans fil avec le dispositif médical implantable (10), dans lequel le dispositif patient (16) détermine l'emplacement géographique (L) du dispositif médical implantable (10) en utilisant le GNSS et/ou les données mobiles.

4. Procédé mis en œuvre par ordinateur selon l'une quelconque des revendications précédentes, dans lequel la condition prédéterminée (C) est remplie si l'emplacement géographique (L) détecté du dispositif médical implantable (10) est classé comme zone à haut risque ou représente un risque spécifique du patient, en particulier en fonction d'une condition de patient spécifiée et/ou si une proximité (P) du dispositif médical implantable (10) vis-à-vis d'un objet à haut risque et/ou d'une personne à haut risque est détectée.

5. Procédé mis en œuvre par ordinateur selon la revendication 4, dans lequel des données d'emplacement géographique (D1) du dispositif médical implantable (10) et/ou des données de proximité (D2) du dispositif médical implantable (10) vis-à-vis de l'objet et/ou de la personne détectés par le dispositif patient (16) sont comparées à des données (D3) sur des zones à haut risque et/ou des risques spécifiques du patient stockés dans une base de données (DB) hébergée sur le dispositif patient (16), le dispositif médical implantable (10) et/ou un serveur distant (18) accessibles par le biais du dispositif patient (16) par l'intermédiaire d'un réseau (20) afin de déterminer si la condition prédéterminée (C) est remplie.

6. Procédé mis en œuvre par ordinateur selon la revendication 5, dans lequel la fonction de diagnostic (12) du dispositif médical implantable (10) est choisie par un professionnel de santé, en particulier un médecin, ou par un algorithme utilisant des données médicales (22) du patient stockées dans le dispositif patient (16), le dispositif médical implantable (10) et/ou le serveur distant (18) étant accessibles par le biais du dispositif patient (16) par l'intermédiaire du réseau (20).

7. Procédé mis en œuvre par ordinateur selon la revendication 5 ou 6, dans lequel le contenu de la base de données (DB) est synchronisé entre le serveur distant (18) et le dispositif médical implantable (10) et/ou le dispositif patient (16) à des intervalles prédéterminés et/ou lorsque la base de données (DB) est mise à jour.

8. Procédé mis en œuvre par ordinateur selon l'une quelconque des revendications précédentes, dans lequel si la condition prédéterminée (C) est remplie, la fonction de diagnostic (12) du dispositif médical implantable (10) est activée et/ou désactivée et/ou la fréquence de transmission (14) et/ou la sélection (15) de l'au moins un paramètre médical (MP) du patient détecté par le dispositif médical implantable (10) est modifiée pendant une période prédéterminée après laquelle la configuration précédente est automatiquement reprise ou jusqu'à réception d'une requête d'annulation générée par l'utilisateur.

9. Procédé mis en œuvre par ordinateur selon l'une quelconque des revendications précédentes, dans lequel la fonction de diagnostic (12) du dispositif médical implantable (10) est une mesure ou un suivi de température, d'impédance, de fonction respiratoire, de son, de posture et/ou de mouvement par des capteurs (10a) du dispositif médical implantable (10).

10. Procédé mis en œuvre par ordinateur selon l'une quelconque des revendications précédentes, dans lequel l'au moins une fonction de diagnostic (12) du dispositif médical implantable (10) est activée et/ou désactivée, et/ou la fréquence de transmission (14) et/ou la sélection (15) de l'au moins un paramètre médical (MP) du patient détecté par le dispositif médical implantable (10) est modifiée pendant une période définie par le patient et/ou le professionnel de santé, en particulier une période de voyage et/ou une durée de quarantaine et/ou pendant une durée automatiquement définie par le dispositif médical implantable (10) ou le dispositif patient (16), en particulier un temps d'incubation.

11. Procédé mis en œuvre par ordinateur selon l'une quelconque des revendications 6 à 10, dans lequel l'au moins un paramètre médical (MP) du patient est surveillé (S3) pendant une période prédéterminée, dans lequel si l'au moins un paramètre médical (MP) du patient est en dehors d'une plage prédéfinie, un message est envoyé depuis le dispositif patient (16) au serveur distant (18) accessible par le professionnel de santé et/ou une fonction thérapeutique (24) du dispositif médical implantable (10) est activée.

12. Procédé mis en œuvre par ordinateur selon l'une quelconque des revendications précédentes, dans lequel si une proximité (P) du dispositif médical implantable (10) au sein d'une plage prédéfinie, en particulier à moins de 1,5 m, vis-à-vis d'une personne infectée est détectée, au moyen d'un traçage de proximité (P) utilisant le Bluetooth, le GNSS et/ou les données mobiles, la condition prédéterminée (C) est remplie et/ou une alerte est envoyée (S4) depuis le dispositif patient (16) au serveur distant (18) accessible par le professionnel de santé.

13. Système (1) destiné à commander un dispositif médical implantable (10) comprenant :
un dispositif patient (16) configuré pour détecter un emplacement géographique (L) du dispositif médical implantable (10) et/ou une proximité (P) du dispositif médical implantable (10) vis-à-vis d'un objet et/ou d'une personne ; et
le dispositif médical implantable (10), dans lequel le dispositif patient (16) est en outre configuré pour commander le dispositif médical implantable (10) par Bluetooth à basse consommation et/ou communication sur bande MICS afin d'activer et/ou de désactiver au moins temporairement au moins une fonction de diagnostic (12) du dispositif médical implantable (10) et/ou de modifier au moins temporairement une fréquence de transmission (14) et/ou une sélection (15) d'au moins un paramètre médical (MP) d'un patient détecté par le dispositif médical implantable (10) si l'emplacement géographique détecté (L) du dispositif médical implantable (10) et/ou la proximité (P) du dispositif médical implantable (10) vis-à-vis de l'objet et/ou de la personne répond à une condition prédéterminée (C).

14. Programme informatique avec un code de programme destiné à réaliser le procédé selon l'une quelconque des revendications 1 à 12 lorsque le programme informatique est exécuté par le système selon la revendication 13.

15. Support de données lisible par ordinateur contenant un code de programme d'un programme informatique destiné à réaliser le procédé selon l'une quelconque des revendications 1 à 12 lorsque le programme informatique est exécuté par le système selon la revendication 13.
